Europäisches Patentamt

European Patent Office

Office européen des brevets

(11) Veröffentlichungsnummer: **0 143 246 B1**

# (12) EUROPÄISCHE PATENTSCHRIFT

(45) Veröffentlichungstag der Patentschrift: **27.11.91**

(51) Int. Cl.⁵: **A61F 2/24**

(21) Anmeldenummer: **84111304.6**

(22) Anmeldetag: **21.09.84**

(54) **Herzklappenprothese.**

(30) Priorität: **23.09.83 DE 8327414 U**

(43) Veröffentlichungstag der Anmeldung:
**05.06.85 Patentblatt 85/23**

(45) Bekanntmachung des Hinweises auf die
Patenterteilung:
**27.11.91 Patentblatt 91/48**

(84) Benannte Vertragsstaaten:
**AT BE CH DE FR GB IT LI LU NL SE**

(56) Entgegenhaltungen:
| | |
|---|---|
| WO-A-82/02829 | DE-U- 8 327 414 |
| FR-A- 2 433 933 | US-A- 3 548 418 |
| US-A- 3 570 014 | US-A- 3 755 823 |
| US-A- 3 983 581 | US-A- 4 084 268 |

(73) Patentinhaber: **Peter Kennedy Pty. Ltd.
4 The Quarter Deck 10 Mounts Bay Road
Crawley 6009, West Australia(AU)**

(72) Erfinder: **Reichart, Bruno
Irmgardstrasse 42
W-8000 München 71(DE)**
Erfinder: **Weinhold, Christian
Hatzfelderweg 13b
W-8000 München 71(DE)**

(74) Vertreter: **Dipl.-Phys.Dr. Manitz Dipl.-Ing.,
Dipl.-W.-Ing. Finsterwald Dipl.-Ing. Grämkow
Dipl.-Chem.Dr. Heyn Dipl.-Phys. Rotermund
Morgan, B.Sc.(Phys.) Robert-Koch-Strasse 1
W-8000 München 22(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Herzklappenprothese mit einer fixierten, einheitlichen, tierischen Aortenklappe nach dem Oberbegriff des Patentanspruchs 1.

Aus der US-A-35 70 014 ist eine Herzklappenprothese mit einem Metallträger bekannt, mit dem eine Herzklappe tierischen Ursprungs vernäht ist. Der im wesentlichen zylindrische Metallträger besitzt drei sich in Axialrichtung erstreckende Pfosten, in denen ebenso wie im Ringbereich der Basis Aussparungen vorgesehen sind.

Aus der WO-A-82/02829 ist es bekannt, zur Herstellung von Herzklappenprothesen das biologische Material von Wildschweinen zu verwenden und dabei die Muskelfasern des "septal cuspae" zu entfernen.

Der prothetische Herzklappenersatz mit biologischen Gewebeklappen zeigt hinsichtlich der Tromboembolierate, der postoperativen Morbidität und der Lebensqualität gute Ergebnisse und ist inzwischen weltweit eine anerkannte Behandlungsmethode. Erfahrung mit porcinen Gewebeklappen liegen seit 1970 vor. Die kürzere Haltbarkeit dieser Prothesen im Vergleich zu mechanischen Ventilen, die vorzeitigen Verkalkungen bei jugendlichen Patienten und die hohen transvalvulären Druckgradienten bei den kleinen Klappengrößen haben jedoch häufig zu Komplikationen und Reoperationen geführt. Letzteres liegt an dem starren Ansatz der rechten Aortentaschenklappe, die dem muskulärem Ventrikelseptum des porcinen Herzens mehrere Millimeter aufsitzt. Nach der Entnahme und Präparation der Klappe werden anhaftende Muskelreste im Fixierungsprozeß mit Glutaraldehyd starr und verhindern somit die freie Beweglichkeit der rechten Taschenklappe.

Um diesem Problem zu begegnen, sind immer neue Methoden entwickelt worden (Verkürzung der Trägerzylinderhöhe, modifizierte Fixierlösungen, Niederdruckfixation), die zu einer Modifizierung der porcinen Bioprothesen führten.

Diese Modifikationen bedeuten jedoch in der Fertigung der Prothesen einen erheblichen Mehraufwand an Technik und Manufaktur, was mit Preissteigerungen verbunden ist.

Die Erfindung beruht auf der Erkenntnis, daß bei den bisher verwendeten porcinen Xenotransplantaten die rechte Taschenklappe an ihrer Basis von ihrem Ansatz am Anulus fibrosus aus gesehen mehrere Millimeter mit dem Muskelwulst des Ventrikelspektrums verwachsen ist. Zur Beseitigung dieses Nachteils versucht man durch vom Anulus fibrosus ausgehende Präparation der rechten Taschenklappe das die freie Beweglichkeit der Taschenklappe störende Muskelgewebe mechanisch, z.B. durch Abschaben zu entfernen. Abgesehen von dem hiermit verbundenen hohen Herstellungsaufwand, läßt sich das störende Muskelgewebe jedoch nicht vollständig entfernen, so daß bei am anschließenden Fixierungsprozeß mit einer 2 %-igen Glutaraldehyd-Lösung die muskulären Anteile starr werden und somit die Taschenbeweglichkeit vom Rande ausgehend behindern. Aus diesem Grunde kann eine derart ausgebildete Herzklappenprothese während des Öffnungszyklus nicht weit genug aufgehen, was einen unerwünscht hohen hämodynamischen Druckgradienten bedingt.

Man hat nun schon versucht, größere porcine Aortenklappen zu verwenden, um ausreichend Material zur Verfügung zu haben, damit das mit Muskelgewebe verwachsene Gewebe der rechten Taschenklappe aus dem Aortenlumen herausgenommen und um den unteren Rand des Trägers herumgeschlungen werden kann. Bei derartigen Prothesen ist das Gewebe der rechten Taschenklappe um den Träger nach außen gezogen, wodurch es bei Langzeitbelastungen häufig zum Gewebebruch mit Bluteinstrom und folgender Verkalkung an der empfindlichen Knickstelle kommt.

Schließlich hat man auch schon versucht, Herzklappenprothesen aus einzelnen Taschenklappen zusammenzusetzen, was jedoch abgesehen von dem außerordentlichen Herstellungsaufwand eine kürzere Lebensdauer erwarten läßt, da die vielen Nahtstellen eine Schwächung bedeuten.

Das Ziel der Erfindung besteht somit darin, eine Herzklappenprothese der eingangs genannten Gattung zu schaffen, bei der die Beweglichkeit insbesondere der rechten Taschenklappe der Beweglichkeit einer menschlichen Herzklappe so nahe wie möglich kommt, ohne daß auf das Entspringen der Taschenklappe aus einem am unteren Rand des Gewebes festgenähten Anulus fibrosus verzichtet werden muß und ohne daß Taschenklappen aus verschiedenen Aortenklappen zusammengestückelt werden müssen.

Zur Lösung dieser Aufgabe sieht die Erfindung die kennzeichnenden Merkmale des Patentanspruchs 1 vor.

Die Erfindung geht also von der Erkenntnis aus, daß für eine durch den Fixierprozeß abgetötete Aortenklappe es wichtig ist, daß sie von einem am Dacron®-Gewebe im Bereich des unteren Randes des Trägerrahmens befestigten Anulus fibrosus entspringt und nicht oder nur in einem die Beweglichkeit kaum beeinträchtigenden Maße mit dem Muskelgewebe des Ventrikelseptums verwachsen ist. Diese Bedingung erfüllt die Aortenklappe eines vorzugsweise männlichen roten und grauen Riesenkänguruhs der Gattung Macropus giganteus und Macropus rufus in idealer Weise.

Zur Herstellung der Aortenklappen werden aus vorfixierten Herzen die Aortenwurzel mit anteiligem Myokard und einem mehrere Zentimeter langen Aortenstumpf herausgeschnitten.

Der Vorteil der Verwendung von fixierten Känguruh-Aortenklappen ist insbesondere darauf zurückzuführen, daß die Aortenwand mit ihren elastischen Fasern und der glatten Muskulatur entlang des Sinus valsalvae bis auf den vorspringenden Muskelwulst übergeht. Erst dann beginnt ein sehr flacher kleiner Anulus fibrosus, der mit einzelnen kleinen Kollagenfaserfingern in der Septummuskulatur verankert ist. Die rechte Taschenklappe entspringt so, ohne dem Muskelwulst aufzuliegen, nahezu frei vom Anulus fibrosus in das Aortenlumen.

Die Erfindung schafft also eine Herzklappenprothese, bei der der Ansatz der rechten Taschenklappe zusammen mit einem wesentlich kleineren Anulus fibrosus in Relation zum Gesamtdurchmesser des Aortenostiums kürzer ist, als dies bei porcinen Xenotransplantaten der Fall ist. Dadurch reduziert sich der nach dem Fixierungsprozeß unbewegliche Anteil der rechten Taschenklappe, was zu einer deutlichen Vergrößerung der Klappenöffnungsfläche während des Öffnungszyklus im Vergleich zu porcinen Xenotransplantaten führt. Bei Verwendung der erfindungsgemäßen Klappen als Xenograft treten also geringere Drucksprünge auf als bei porcinen Gewebeklappen. So kann gerade bei kleinen Klappengrößen eine wesentliche Funktionsverbesserung erreicht werden. Känguruh-Aortenklappen können in den Größen 17 bis 31 mm hergestellt werden. Sie lassen sich als Klappenersatz in allen vier Positionen des Herzens einsetzen, also z.B. als Aorten- oder Mitralklappe verwenden.

Der Trägerrahmen besteht vorzugsweise aus einem thermisch sehr stabilen Kunststoff, z.B. einem Acetyl-Copolymer-Harz (Delrin®). Bei dem den Träger überziehenden Gewebe handelt es sich im allgemeinen um ein Doppelstrick-Polyesterfabrikat.

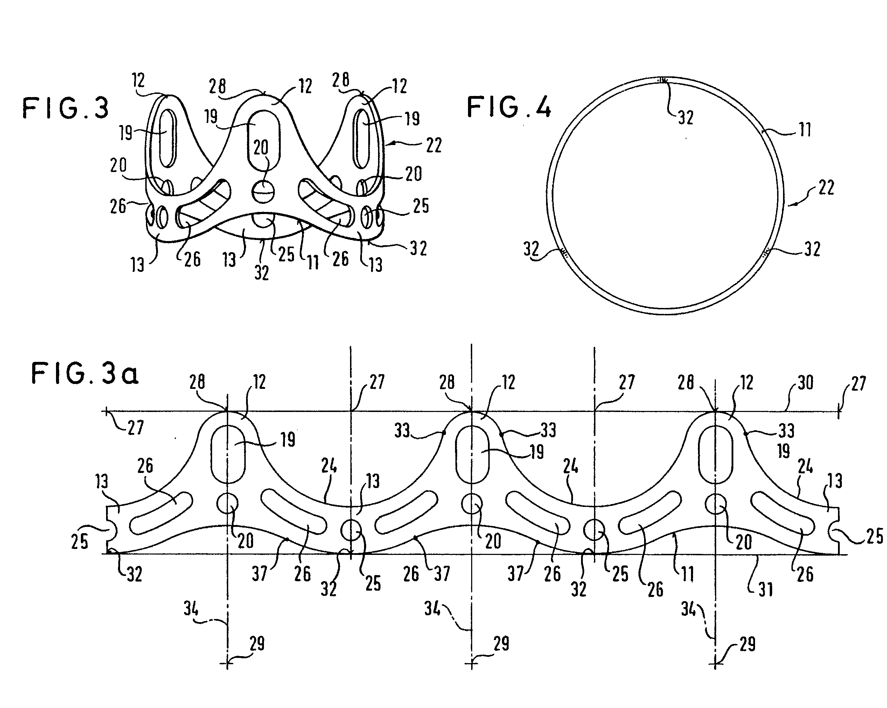

Um die Vorteile einer fixierten Känguruh-Aortenklappe und insbesondere deren günstiges Öffnungsverhältnis voll zur Wirkung kommen zu lassen, wird der Trägerrahmen in besonders vorteilhafter Weise nach einem oder mehreren der Patentansprüche 2 bis 11 ausgebildet. Die besonders bevorzugten Dimensionierungsangaben beziehen sich dabei auf einen in eine Ebene abgewickelten Trägerrahmen (Fig. 3a).

Die Breite der für die Anordnung der Kommissuren vorgesehenen Pfosten beträgt erfindungsgemäß etwa 1/4 des Außendurchmessers des Trägerrahmens; die äquidistanten Kommissuren-Pfosten sind erfindungsgemäß also so breit ausgebildet, daß etwa eine 30°-Toleranz bei der Anordnung der Kommissuren vorliegen kann. Bei Känguruh-Aortenklappen schwankt die Anordnung der Kommissuren demgegenüber nur um einen Winkel von etwa 15°.

Nach einer vorteilhaften Ausführung der Erfindung ist weiter vorgesehen, daß der Trägerrahmen gegenüber den bisher für porcine Klappen verwendeten Trägerrahmen deutlich kürzere Kommissuren-Pfosten aufweist. Auch das Gesamtprofil ist axial niedriger.

Der von den Pfosten abgewandte Eintrittsrand ist derart schwach wellenförmig ausgebildet, daß an ihn ein flacher Mitral-Nahtring ebenso angebracht werden kann wie ein bogenförmiger Aorten-Nahtring. Besonders vorteilhaft kann die erfindungsgemäße Prothese auch in eine Gefäßprothese mit eingebauter Herzklappe integriert werden.

Besonders wichtig sind die zahlreichen Löcher, Bohrungen und Schlitze im Trägerrahmen, welche praktisch in allen Bereichen des Trägerrahmens vorgesehen sind, so daß nicht nur die elastischen Eigenschaften des Trägerrahmens wesentlich verbessert sind, sondern auch viele Befestigungsmöglichkeiten für das in Form eines Schlauches vorliegende, den Trägerrahmen allseitig überdeckende Dacron-Gewebe® vorhanden sind.

Die Breite des Ringbereiches ist so gewählt, daß ein maximaler Raum für eine freie Beweglichkeit der Taschenklappen vorhanden ist.

Die Erfindung wird im folgenden beispielsweise anhand der Zeichnung beschrieben; in dieser zeigt

Fig. 1    eine Seitenansicht einer erfindungsgemäßen Herzklappenprothese etwa im Maßstab 3 : 1,

Fig. 2    eine Ansicht des Gegenstandes der Fig. 1 von unten, jedoch um 180° um die Mittelachse gedreht,

Fig. 3    eine Seitenansicht des bei der Herzklappenprothese nach den Fig. 1 und 2 verwendeten Trägerrahmens,

Fig. 3a   eine Abwicklung des Trägerrahmens nach Fig. 3 in die Zeichnungsebene und

Fig. 4    eine Ansicht des Trägerrahmens nach Fig. 3 von unten.

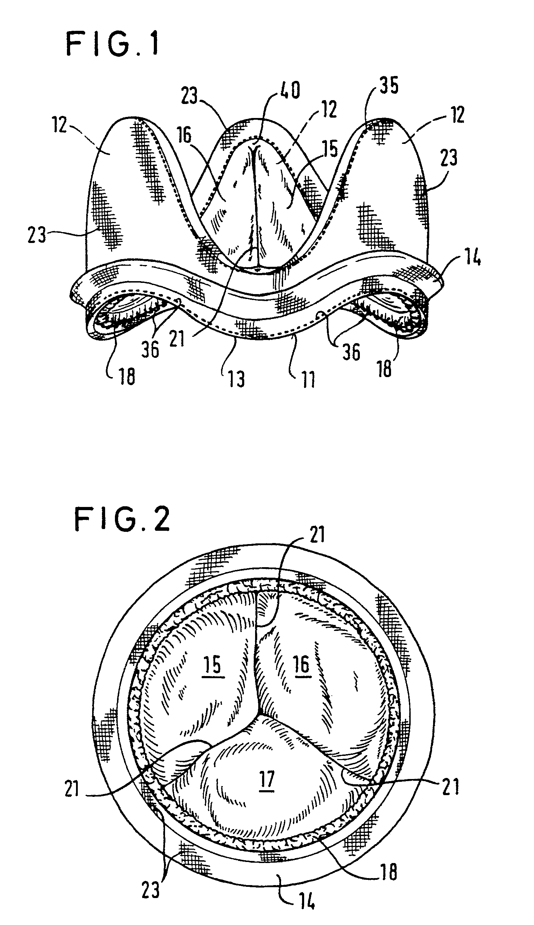

Nach den Fig. 1 und 2 weist ein innen und außen mit einem Polyester-, z.B. Dacron®-Gewebe 23 überzogener Trägerrahmen 22 (Fig. 3, 4) aus einem Acetylharz, z.B. Delrin® eine im wesentlichen kreiszylindrische Form auf. Am in Fig. 1 unteren Rand 11 ist das Gewebe 23 durch Faltenbildung zu einem Nahtring 14 geformt, in dem ein den unteren Rand 11 des Trägerrahmens 22 umgebender elastischer Stützring untergebracht sein kann. Der Nahtring 14 dient zum Vernähen der Herzklappenprothese mit dem menschlichen Gewebe, z.B. der Aorta. Nach den Fig. 3, 3a und 4 besteht der Trägerrahmen 22 aus einem zu einer Kreiszylinderform aufgewickelten bandförmigen Material, dessen Stärke etwa 1/40 des Außendurchmessers des Trägerrahmens 22 beträgt.

Wie insbesondere aus Fig. 3 und deren ebener Abwicklung nach Fig. 3a hervorgeht, weist das bandförmige Material einen leicht wellenförmig geschwungenen Ringbereich 13 mit drei vollen, jeweils einen Wellenberg und ein Wellental aufweisenden Wellen auf, wobei sich im Bereich jedes Wellenberges ein Pfosten 12 axial in der späteren Öffnungsrichtung der Taschenklappen erstreckt.

In jedem Pfosten 12 befinden sich axial verlaufende Langlöcher 19, welche die Form zweier durch gerade Linien verbundener Halbkreise aufweisen, deren Radius das 0,064-fache des Außendurchmessers des Trägerrahmens 22 beträgt. Der Abstand der beiden Mittelpunkte der Halbkreise liegt bei 1/10 des Außendurchmessers des Trägerrahmens 22.

Im Bereich ihrer Scheitel 28 sind die Pfosten 12 entsprechend dem oberen Halbkreis der Langlöcher 19 abgerundet, so daß zwischen den Langlöchern 19 und der oberen Berandung der Pfosten 12 Material mit einer Breite verbleibt, die gleich 1/16 des Außendurchmessers des Trägerrahmens 22 ist.

Zwischen zwei benachbarten Pfosten 12 ist der Ringbereich 13 oben durch einen oberen Rand 24 begrenzt, der die Form eines konkaven Kreisbogens mit seinem Krümmungsmittelpunkt 27 in der Mitte der Verbindungslinie 30 zwischen den Scheiteln 28 benachbarter Pfosten 12 besitzt. Der obere Rand 24 hat also in der Abwicklung nach Fig. 3a annähernd Halbkreisform und geht über einen Umkehrpunkt 33 in den den Scheitel 28 aufweisenden Bereich der Pfosten 12 über.

Auf der vom Scheitel 28 axial abgewandten Seite des Langloches 19 befindet sich im Ringbereich 13 ein kreisförmiges Loch 20, dessen Durchmesser um einen Faktor 0,093 kleiner als der Außendurchmesser des Trägerrahmens 22 ist. Das Loch 20 befindet sich in der Mitte zwischen dem Langloch 19 und dem unteren Rand 11 des Ringbereiches 13.

Der untere Rand 11 des Trägerrahmens 22 ist im Bereich der Pfosten 12 konkav kreisförmig gekrümmt, wobei der Krümmungsmittelpunkt 29 auf der Mittelachse 34 des Langloches 19 in deutlichem Abstand von der Verbindungslinie 31 zwischen benachbarten Scheiteln 32 des wellenförmig geformten unteren Randes 11 liegt, und zwar auf der von dem Pfosten 12 abgewandten Seite der Verbindungslinie 31. Bevorzugt ist der Abstand des Krümmungsmittelpunktes 29 von der Verbindungslinie 31 etwa gleich dem halben Außendurchmessers des Trägerrahmens 22. Der Radius des Kreisbogens um den Krümmungsmittelpunkt 29 ist um etwa 50 % größer als der Radius des Kreisbogens um den oberen Krümmungsmittelpunkt 27 und beträgt zweckmäßig das 0,6-fache des Außendurchmessers des Trägerrahmens 22. Die Krümmungsmittelpunkte 27 und 29 sind in Umfangsrichtung um einen Winkel von 60° gegeneinander versetzt.

Auch die Scheitel 28 und 32 sind in Umfangsrichtung um einen Winkel von 60° gegeneinander versetzt. Der kreisbogenförmige Teil des unteren Randes 11 mit dem Krümmungsmittelpunkt 29 geht über Wendepunkte 37 in im Bereich des Scheitels 32 liegende, kreisförmig konvex gekrümmte Teile mit annähernd gleichem Krümmungsradius wie der Bereich mit dem Krümmungsmittelpunkt 29 über.

In der Mitte des bandförmigen Ringbereiches 13 befindet sich an derjenigen Umfangsstelle, wo die Scheitel 32 vorgesehen sind, jeweils eine Kreisbohrung 25 mit einem Durchmesser, der um einen Faktor 0,064 geringer als der Außendurchmesser des Trägerrahmens 22 ist.

Beidseitig an diese Bohrung 25 schließt sich mit einem geringen Abstand etwa gleich dem Durchmesser der Bohrung 25 jeweils ein Längsschlitz 26 an, der entsprechend dem kreisförmigen konkaven Teil des oberen Randes 24 gekrümmt ist und parallel zu diesem Teil des oberen Randes 14 verläuft. Die Längsschlitze 26 erstrecken sich etwa bis zum Beginn der Pfosten 12 und enden in einem Abstand von den Langlöchern 19 bzw. dem Loch 20, der in etwa der Breite der Langlöcher 19 entspricht. Im Bereich der Scheitel 32 verlaufen die Ränder 11, 24 im wesentlichen parallel zueinander.

Aufgrund der erfindungsgemäßen, nur leicht gewellten Form des Ringbereiches 13 und der axial relativ kurzen Pfosten 12 sowie der erfindungsgemäßen Durchsetzung des Trägerrahmens 22 mit Löchern, Bohrungen und Schlitzen eignet sich der Trägerrahmen nach dem Anbringen des Dacron®-Gewebes 23 gemäß den Fig. 1 und 2 optimal zur Anbringung einer Känguruh-Aortenklappe mit dem daran noch befindlichen Aortenstumpf 40. Innerhalb dieses Trägerrahmens 22 können sich die Taschenklappen 15, 16, 17 (Fig. 1 und 2) einwandfrei in die volle Öffnungsstellung bewegen und auch wieder ungehindert schließen. Der Trägerrahmen 22 weist aufgrund der erfindungsgemäßen Ausbildung außerdem ein hervorragendes elastisches Verhalten auf, welches weitgehend dem elastischen Verhalten der Aorta oder des Herzteils entspricht, in den die Herzprothese implantiert wird. Die Herzklappenprothese beeinträchtigt also die natürlichen Verformungsvorgänge der mit ihr in Verbindung stehenden Teile des menschlichen Gewebes nicht in schädlicher Weise.

Die zahlreichen Durchbrechungen in dem Kunststoff-Trägerrahmen 22 gewährleisten darüber hinaus eine einwandfreie Befestigungsmöglichkeit für das darüber zu ziehende Dacron®-Gewebe 23.

Nach der Fertigstellung des aus Fig. 3 ersichtlichen Trägerrahmens 22 wird zunächst das Dacron Gewebe in üblicher Weise darüber gezogen und festgenäht. Anschließend wird dann in den mit dem Gewebe 23 überzogenen Trägerrahmen 22 nach den Fig. 1 und 2 die durch Fixieren abgetötete und haltbar gemachte, noch einen Aortenstumpf 40 aufweisende Aortenklappe eines Känguruhs eingesetzt, welche aus dem Anulus fibrosus 18 und den aus diesen entspringenden drei Taschenklappen 15, 16, 17 besteht, und zwar der rechten Taschenklappe 15, der a-coronaren Taschenklappe 16 und der linken Taschenklappe 17. Die Taschenklappen werden gelegentlich auch als Segel bezeichnet.

Zur Montage wird der Anulus fibrosus 18 am unteren Rand des Trägerrahmens 22 angeordnet und der an den Anulus fibrosus 18 anschließende Aortenstumpf 40, der oben entsprechend dem Verlauf des Randes 24 (Fig. 3a) ausgeschnitten ist, innen an dem Ringbereich 13 und den Pfosten 12 hochgeführt sowie im Bereich des oberen Randes 24 (Fig. 3,3a) mit dem Gewebe 23 vernäht, wobei eine Naht 35 entsteht. Nach Fig. 2 ist der Anulus fibrosus 18 mit dem Nahtring 14 vernäht,wodurch sich eine weitere Naht 36(Fig. 1) ergibt.

Im Bereich der rechten Taschenklappe 15 ist am Anulus fibrosus 18 noch ein nicht dargestellter kleiner Rest von Muskelgewebe des Ventrikelseptums vorhanden. Aufgrund der Verwendung einer Känguruh-Aortenklappe ist dieser Muskelgeweberest jedoch in radialer Richtung so klein dimensioniert und auch in seiner Stärke so unbedeutend, daß er die freie Beweglichkeit der rechten Taschenklappe 15 während des Öffnungsvorgangs der Aortenklappe praktisch nicht behindert.

Nach der Implantation in einen menschlichen Körper arbeitet die erfindungsgemäße Herzklappenprothese wie folgt:
Wenn auf der in Fig. 1 unteren Seite der Prothese, wo sich die linke Herzkammer befindet, ein Überdruck entsteht, klappen die Taschenklappen 15, 16, 17 nach der Art eines Ventils nach oben und geben aufgrund der erfindungsgemäßen Ausbildung einen Öffnungsquerschnitt frei, der mindestens 70, im allgemeinen jedoch 75 % und unter Umständen sogar 80 % der Querschnittsfläche des Aortenlumens beträgt. Auf diese Weise wird in erwünschtem Maße der zwischen den beiden Seiten der Herzklappenprothese auftretende Druckgradient im Öffnungsfall minimal gehalten.

Kehren sich die Druckverhältnisse um, kehren die Taschenklappen 15, 16, 17 in die aus den Figuren 1 und 2 ersichtliche Ruhelage zurück, wobei sie entlang der Linien Kommissuren 21 in Fig. 2 in eine abdichtende Berührung miteinander kommen, so daß jeder Durchfluß von Blut in der entgegengesetzten Richtung vermieden ist.

**Patentansprüche**

1. Herzklappenprothese mit einer fixierten, einheitlichen, tierischen Aortenklappe, welche an einem mit einem Gewebe überzogenen Trägerrahmen mit im wesentlichen kreiszylindrischem Querschnitt angeordnet ist, welcher einen sich axial erstreckenden Ringbereich und drei sich in gleichmäßigem Winkelabstand vom Ringbereich axial erstreckende, abgerundete Pfosten aufweist, wobei das Gewebe an der von den Pfosten abgewandten Stirnseite zu einem Nahtring ausgeformt ist, wobei der Anulus fibrosus, aus dem die Taschenklappen entspringen und in dessen Bereich sich Muskelgewebe des Ventrikelseptums befindet, im wesentlichen in der Ebene des Nahtringes liegt und dort mit dem Gewebe vernäht ist, und wobei die entsprechend der Form des Trägerrahmens ausgeschnittene Aortenwand innerhalb des Ringbereichs und der Pfosten des Trägerrahmens angeordnet und entlang des auf der Seite der Pfosten vorliegenden Randes des Trägerrahmens mit dem Gewebe vernäht ist,

dadurch **gekennzeichnet,**

daß die Aortenklappe die Aortenklappe eines ausgewachsenen Känguruhs ist, so daß die rechte Taschenklappe (15) ebenso wie die übrigen beiden Taschenklappen (16, 17) frei vom Anulus fibrosus (18) in den Klappenhohlraum vorsteht, ohne durch Verwachsung noch mit Muskelgewebe des Ventrikelseptums verbunden zu sein.

2. Herzklappenprothese nach Anspruch 1,
dadurch **gekennzeichnet,**
daß der Ringbereich (13) des elastisch biegsamen, eine Anzahl von Durchbrechungen aufweisenden Trägerrahmens (22) axial leicht wellenförmig geschwungen ist und drei volle Wellen umfaßt,
daß die Pfosten (12) des Trägerrahmens (22) sich bis in den Ringbereich (13) hineinerstreckende Langlöcher (19) mit axial ausgerichteter Längsachse aufweisen, wobei das Verhältnis der Länge der Langlöcher (19) zum Außendurchmesser des Trägerrahmens (22) vorzugsweise 0,2 bis 0,3 und insbesondere 0,25 beträgt.

3. Herzklappenprothese nach Anspruch 2,
dadurch **gekennzeichnet,**
daß die Langlöcher (19) die Form durch gerade Linien verbundener Halbkreise haben, deren Radius zweckmäßig 1/14 bis 1/17 und insbesondere 1/15 bis 1/16 des Außendurchmessers des Trägerrahmens (22) beträgt.

4. Herzklappenprothese nach einem der vorhergehenden Ansprüche,
dadurch **gekennzeichnet,**
daß der Ringbereich (13) dort, wo die Pfosten (12) vorgesehen sind, ein vorzugsweise kreisförmiges Loch (20) aufweist, dessen Durchmesser 1/10 bis 1/13 und insbesondere 1/11 bis 1/12 des Außendurchmessers des Trägerrahmens (22) beträgt.

5. Herzklappenprothese nach einem der vorhergehenden Ansprüche,
dadurch **gekennzeichnet,**
daß Ringbereich (13) in der Mitte zwischen je zwei Pfosten (12) eine mittige Bohrung (25) aufweist, deren Durchmesser vorzugsweise 1/14 bis 1/17 und insbesondere 1/15 bis 1/16 des Außendurchmessers des Trägerrahmens (22) beträgt.

6. Herzklappenprothese nach einem der vorhergehenden Ansprüche,
dadurch **gekennzeichnet,**
daß der Rand (24) des Trägerrahmens (22) auf der Seite der Pfosten (12) zwischen den Pfosten (12) konkav kreisförmig verläuft, wobei der Radius vorzugsweise 1/2 bis 1/3 und insbesondere etwa das 0,4-fache des Außendurchmessers des Trägerrahmens (22) beträgt und der Krümmungsmittelpunkt (27) zweckmäßig in der Mitte der Verbindungslinie (30) zwischen den Scheiteln (28) zweier benachbarter Pfosten (12) liegt.

7. Herzklappenprothese nach einem der vorhergehenden Ansprüche,
dadurch **gekennzeichnet,**
daß der Ringbereich (13) zwischen jedem Pfosten (12) und der Mitte zwischen zwei Pfosten (12) einen der Krümmung des Ringbereiches (13) folgenden Längsschlitz (26) mit einer bevorzugten Breite gleich 1/14 bis 1/17, insbesondere 1/15 bis 1/16 des Außendurchmessers des Trägerrahmens (22) aufweist, wobei jeder Längsschlitz (26) von der Mitte der Pfosten (12) einen peripheren Abstand hat, der vorzugsweise etwa 1/7 bis 1/9, insbesondere 1/8 des Außendurchmessers des Trägerrahmens (22) beträgt, wobei jeder Längsschlitz (26) gegebenenfalls von der mittleren Bohrung (25) einen peripheren Abstand hat, der vorzugsweise 1/14 bis 1/17, insbesondere 1/15 bis 1/16 des Außendurchmessers des Trägerrahmens (25) beträgt, und wobei die Breite des Ringbereiches (13) neben der mittleren Bohrung (25) bzw. den Längsschlitzen (26) vorzugsweise 1/14 bis 1/17, insbesondere 1/15 bis 1/16 des Außendurchmessers des Trägerrahmens (22) beträgt.

8. Herzklappenprothese nach einem der vorhergehenden Ansprüche,
dadurch **gekennzeichnet,**

daß der Ringbereich (13) außerhalb der Pfosten (12) eine Breite 1/6 bis 1/4 und insbesondere etwa 1/5 des Außendurchmessers des Trägerrahmens (22) besitzt.

9. Herzklappenprothese nach einem der vorhergehenden Ansprüche,
dadurch **gekennzeichnet,**
daß der Rand (11) auf der von den Pfosten (12) abgewandten Seite im Bereich der Pfosten (12) konkav kreisförmig verläuft, wobei der Radius vorzugsweise das 0,5- bis 0,7- und insbesondere das 0,6-fache des Außendurchmessers des Trägerrahmens (22) beträgt und der Krümmungsmittelpunkt (29) zweckmäßig auf der Mittelachse der Pfosten (12) liegt und der Abstand jedes Krümmungsmittelpunktes (29) von der Verbindungslinie (31) zwischen den von den Pfosten (12) abgewandten Scheiteln (32) zweckmäßig das 0,4- bis 0,5- und insbesondere das 0,48-fache des Außendurchmessers des Trägerrahmens (22) beträgt.

10. Herzklappenprothese nach einem der vorhergehenden Ansprüche,
dadurch **gekennzeichnet,**
daß die axiale Länge des Trägerrahmens (22) das 0,58- bis 0,62- und insbesondere etwa das 0,6-fache seines Außendurchmessers beträgt.

11. Herzklappenprothese nach einem der vorhergehenden Ansprüche,
dadurch **gekennzeichnet,**
daß die Materialstärke des Trägerrahmens (22) 1/35 bis 1/45 und insbesondere etwa 1/40 des Außendurchmessers des Trägerrahmens beträgt.

## Claims

1. Heart valve bioprothesis with a fixed, integral aortic valve of animal origin, which is positioned in a fabric-covered supporting frame having a substantially right cylindrical cross-section, the supporting frame comprising an axially extending annular region and three rounded posts axially extending from the annular region at equal angular intervals; wherein the fabric is shaped as a suture ring at the end face remote from the posts; wherein the annulus fibrosus, from which the pocket leaflets originate and in the area of which muscular tissue of the venticular septum is to be found, is placed essentially in the plane of the suture ring and is sutured there to the fabric; and wherein the aortic wall which is cut according to the shape of the supporting frame is arranged within the annular region and the posts of the supporting frame and is sutured to the fabric along the border of the supporting frame present on the side of the posts, characterised in that the aortic valve is the aortic valve of a fullgrown adult kangaroo such that the right leaflet (15) as well as the two other leaflets (16, 17) project unimpeded from the annulus fibrosus (18) into the valve cavity, without being coalesced with the muscular tissue of the ventricular septum.

2. Heart valve prothesis in accordance with claim 1, wherein the annular region (13) of the resiliently flexible supporting frame (22) which comprises a number of perforations is axially slightly undulated and comprises three complete waves; in that the posts (12) of the supporting frame (22) are provided with oblong perforations (19) extending up to the annular region (13) and having axially directed longitudinal axes, wherein the ratio of the length of the oblong perforations (19) to the external diameter of the supporting frame (22) lies in the range from 0.2 to 0.3, and in particular 0.25.

3. Heart valve prothesis in accordance with claim 2, wherein the shape of each of the oblong perforations (19) is that of two semicircles connected by straight lines, the radius of said semicircles lying expediently in the range from 1/14th to 1/17th, and in particular from 1/15th to 1/16th, of the external diameter of the supporting frame (22).

4. Heart valve prothesis in accordance with one of the preceding claims, wherein the annular region (13) comprises, where the posts (12) are provided, a preferably circular hole (20), the diameter of which is 1/10th to 1/13th, and in particular 1/11th to 1/12th, of the external diameter of the supporting frame (22).

5. Heart valve prothesis in accordance with one of the preceding claims, wherein the annular region (13) has a central bore (25) midway between each adjacent pair of posts (12), the diameter of each said central bore being 1/14th to 1/17th, and in particular 1/15th to 1/16th, of the external diameter of the supporting frame (22).

6. Heart valve prothesis in accordance with one of the preceding claims, wherein the border (24) of the supporting frame (22) is shaped in a concave circular manner between the posts (12) on the side of the posts (12), the radius lying preferably in the range from 1/2 to 1/3 times and in particular about 0.4 times the external diameter of the supporting frame (22) and the centre of curvature (27) being expediently positioned at the center of the connecting line (30) between the crests (28) of two adjacent posts (12).

7. Heart valve prothesis in accordance with one of the preceding claims, wherein the annular region (13) is provided between each post (12) and the middle between two posts (12) with an elongate slot (26) following the curvature of the annular region (13) and having a width lying preferably in the range from 1/14th to 1/17th, in particular in the range from 1/15th to 1/16th, of the external diameter of the supporting frame (22), wherein each elongate slot (26) is spaced from the middle of the posts (12) by a peripheral distance lying preferably in the range from 1/7th to 1/9th, and in particular 1/8th, of the external diameter of the supporting frame (22), wherein each elongate slot (26) has optionally a peripheral distance from the central bore (25) preferably in the range of 1/14th to 1/17th, and in particular 1/15th to 1/16th, of the external diameter of the supporting frame (22), and wherein the width of the annular region (13) near the central bore (25) or the elongate slot (26) lies preferably in the range 1/14th to 1/17th, and in particular 1/15th to 1/16th, of the external diameter of the supporting frame (22).

8. Heart valve prothesis in accordance with one of the preceding claims, wherein the annular region (13) has outwardly of the posts (12) a width lying in the range of 1/6th to 1/4th, and in particular about 1/5th, of the external diameter of the supporting frame (22).

9. Heart valve prothesis in accordance with one of the preceding claims, wherein the border (11) on the side remote from the posts (12) in the region of the posts (12) is shaped so that it is circularly concave, the radius lying preferably in the range of 0.5 to 0.7 times, and in particular 0.6 times, the external diameter of the supporting frame (22), and the centre of curvature (29) lying expediently on the central axis of the posts (12), and the distance between each center of curvature (29) and the line (31) connecting the crests (32) remote from the posts (12) lies expediently in the range of 0.4 to 0.5 times, and in particular 0.48 times the external diameter of the supporting frame (22).

10. Heart valve prothesis in accordance with one of the preceding claims, wherein the axial length of said supporting frame (22) lies in the range of 0.58 to 0.62, and in particular about 0.6, times its external diameter.

11. Heart valve prothesis in accordance with one of the preceding claims, wherein the material thickness of said supporting frame (22) lies in the range from 1/35th to 1/45th, and in particular 1/40th of the external diameter of the supporting frame.

## Revendications

1. Prothèse de valvule cardiaque comprenant une valvule d'aorte animale fixée et unitaire qui est montée sur un cadre support recouvert d'un tissu et ayant une section essentiellement en forme de cylindre droit, le cadre comprenant une zone annulaire qui s'étend axialement et trois poteaux arrondis qui s'étendent axialement depuis la zone annulaire à des distances angulaires égales, dans laquelle le tissu est conformé en une suture annulaire sur la face d'extrémité éloignée des poteaux, dans laquelle l'anneau fibreux, à partir duquel partent les clapets en poche et dans la région duquel se trouve le tissu musculaire du septum ventriculaire, est disposé essentiellement dans le plan de la suture annulaire et est suturé au tissu à cet endroit, et dans laquelle la paroi de l'aorte, qui est découpée suivant la forme du cadre support, est disposée à l'intérieur de la région annulaire et des poteaux du cadre support et est suturée au tissu le long du bord du cadre support présent sur le côté des poteaux, caractérisée en ce que la valvule d'aorte est la valvule d'aorte d'un kangourou adulte, de sorte que le clapet en poche droit (15) ainsi que les deux autres clapets en poche (16, 17) se projettent librement depuis l'anneau fibreux (18) dans la cavité de valvule sans être reliés avec le tissu musculaire du septum ventriculaire en raison de croissance tissulaire.

2. Prothèse de valvule cardiaque selon la revendication 1, caractérisée en ce que la région annulaire (13) du cadre support (22) élastiquement flexible qui comporte un nombre d'ouvertures, est formée axialement avec de légères ondulations et comporte trois ondulations entières, et en ce que les poteaux (12) du cadre support (22) comportent des trous oblongs (19) qui s'étendent jusque dans la région annulaire (13) et ont des axes longitudinaux dirigés axialement, dans laquelle le rapport de la longueur des trous oblongs (19) au diamètre extérieur du cadre support (22) est de préférence de 0.2 à 0.3, et en particulier 0.25.

3. Prothèse de valvule cardiaque selon la revendication 2, caractérisée en ce que les trous oblongs (19) ont la forme de demi-cercles reliés par des lignes droites, le rayon des demi-cercles étant convenablement de 1/14e à 1/17e, et en particulier 1/15e à 1/16e, du diamètre extérieur du cadre support (22).

4. Prothèse de valvule cardiaque selon l'une des revendications précédentes, caractérisée en ce que la région annulaire (13) comporte, à l'endroit où sont prévus les poteaux (12), un trou de (20) préférence circulaire dont le diamètre est de 1/10e à 1/13e, et en particulier 1/11e à 1/12e, du diamètre extérieur du cadre support (22).

5. Prothèse de valvule cardiaque selon l'une des revendications précédentes, caractérisée en ce que la région annulaire (13) comporte un trou central (25) au milieu entre chaque paire de poteaux (12), le diamètre de ce trou étant de préférence de 1/14e à 1/17e, et en particulier de 1/15e à 1/16e, du diamètre extérieur du cadre support (22).

6. Prothèse de valvule cardiaque selon l'une des revendications précédentes, caractérisée en ce que la bordure (24) du cadre support (22) s'étend en forme de cercle concave entre les poteaux (12) du côté des poteaux (12), le rayon étant de préférence de 1/2 à 1/3, et en particulier environ 0.4 fois le diamètre extérieur du cadre support (22), et le centre de courbure (27) étant situé de façon appropriée au centre de la ligne de liaison (30) entre les crêtes (28) de deux poteaux (12) voisins.

7. Prothèse de valvule cardiaque selon l'une des revendications précédentes, caractérisée en ce que la région annulaire (13) comporte, entre chaque poteau (12) et le milieu entre deux poteaux (12) une fente allongée (26) qui suit la courbure de la région annulaire (13) et dont la largeur est de préférence de 1/14e à 1/17e, et en particulier 1/15e à 1/16e du diamètre extérieur du cadre support (22), dans laquelle chaque fente allongée (26) présente par rapport au milieu des poteaux (12) une distance périphérique qui est de préférence de 1/7e à 1/9e, en particulier 1/8e du diamètre extérieur du cadre support (22), dans laquelle chaque fente allongée (26) est située en option à une distance périphérique du trou central (25) qui est de préférence de 1/14e à 1/17e, et en particulier de 1/15e à 1/16e, du diamètre extérieur du cadre support (22), et dans laquelle la largeur de la région annulaire (13) au voisinage du trou central (25), ou des fentes allongées (26), est de préférence de 1/14e à 1/17e, et en particulier de 1/15e à 1/16e, du diamètre extérieur du cadre support (22).

8. Prothèse de valvule cardiaque selon l'une des revendications précédentes, caractérisée en ce que la région annulaire (13) a, à l'extérieur des poteaux, une largeur qui est de 1/6e à 1/4, et en particulier environ 1/5e, du diamètre extérieur du cadre support (22).

9. Prothèse de valvule cardiaque selon l'une des revendications précédentes, caractérisée en ce que la la bordure (11) du côté éloigné par rapport aux poteaux (12) est conformée de manière circulaire concave dans la région des poteaux (12), le rayon étant de préférence de 0.5 à 0.7, et en particulier 0.6 fois le diamètre extérieur du cadre support (22) et le centre de courbure (29) étant situé de manière appropriée sur l'axe médian des poteaux (12), et la distance entre chaque centre de courbure (29) et la ligne de liaison (31) entre les crêtes (32) éloignées des poteaux (12) étant de façon appropriée de 0.4 à 0.5, et en particulier 0.48 fois le diamètre extérieur du cadre support (22).

10. Prothèse de valvule cardiaque selon l'une des revendications précédentes, caractérisée en ce que la longueur axiale du cadre support (22) est de 0.58 à 0.62, et en particulier environ 0.6 fois son diamètre extérieur.

11. Prothèse de valvule cardiaque selon l'une des revendications précédentes, caractérisée en ce que l'épaisseur du matériau du cadre support (22) est de 1/35e à 1/45e, et en particulier environ 1/40e du diamètre extérieur du cadre support.

# FIG.1

# FIG.2

FIG.3

FIG.4

FIG.3a

EP 0 143 246 B1